# EUROPEAN PATENT APPLICATION

(11) **EP 4 425 166 A1**
(43) Date of publication of application: **04.09.2024**
(21) Application number: 22886782.6
(22) Date of filing: 18.10.2022
(51) Int. Cl.: G01N 27/416

(54) **TREATMENT DEVICE USING REACTION SYSTEM CONTAINING OXIDOREDUCTASE AND CO-ENZYME, AND METHOD FOR CONTROLLING SAME**

(30) Priority: 28.10.2021 JP 2021176185
(71) Applicant: Panasonic Intellectual Property Management Co., Ltd., Kadoma-shi, Osaka 571-0057 (JP)
(72) Inventor: TSUJI Kiyotaka, Kadoma-shi Osaka 571-0057 (JP)
(74) Representative: Grünecker Patent- und Rechtsanwälte PartG mbB
(86) International application number: PCT/JP2022/038667
(87) International publication number: WO 2023/074454

(57) **Abstract**

The present disclosure provides a method for controlling a treatment apparatus having advantages from the viewpoint of dealing with a change in a component contained in a treatment target while using a reaction system containing a redox enzyme and a coenzyme. The method for controlling a treatment apparatus according to the present disclosure includes acquiring of a first relationship R1 between a first voltage value and a first current value corresponding to the first voltage value by applying a voltage between a first electrode (11) and a second electrode (12) in a state where a treatment liquid (2) does not contain the redox enzyme or the coenzyme. The controlling method includes acquiring of a second relationship R2 between a second voltage value and a second current value corresponding to the second voltage value by applying a voltage between the first electrode (11) and the second electrode (12) in a state where the treatment liquid (2) contains the redox enzyme and the coenzyme. The controlling method determines a voltage Vt for treating the treatment liquid (2) and a target current Iₐ serving as an indicator of completion of treatment of the treatment liquid (2) in accordance with the first relationship R1 and the second relationship R2.

## Description

### Technical Field

The present disclosure relates to a method for controlling a treatment apparatus using a reaction system containing a redox enzyme and a coenzyme and to a treatment apparatus using a reaction system containing a redox enzyme and a coenzyme.

### Background Art

Technology known in the related art uses a reaction system containing a redox enzyme and a coenzyme.

For example, PTL 1 describes a method for measuring glucose concentration by utilizing a reaction system containing an enzyme and an electron transport mediator. In the method, glucose dehydrogenase (CyGDH) to which cytochrome C is bonded is used as the enzyme, and a Ru compound is used as the electron transport mediator. In a glucose sensor including a reagent layer composed of the Ru compound and CyGDH in combination, a reaction with glucose is completed, and an end point of glucose concentration measurement is reached in a short time.

### Citation List

### Patent Literature

PTL 1: International Publication No. 03/106702

### Summary of Invention

### Technical Problem

The present disclosure provides a method for controlling a treatment apparatus having advantages from the viewpoint of dealing with a change in a component contained in a treatment target while using a reaction system containing a redox enzyme and a coenzyme.

### Solution to Problem

The present disclosure provides a method for controlling a treatment apparatus using a reaction system containing a redox enzyme and a coenzyme,
the treatment apparatus including a reaction tank storing a treatment liquid, a first electrode disposed in contact with the treatment liquid in the reaction tank, and a second electrode disposed in contact with the treatment liquid in the reaction tank,
the method including:
   acquiring a first relationship between a first voltage value and a first current value corresponding to the first voltage value by applying a voltage between the first electrode and the second electrode in a state where the treatment liquid does not contain the redox enzyme or the coenzyme,
   acquiring a second relationship between a second voltage value and a second current value corresponding to the second voltage value by applying a voltage between the first electrode and the second electrode in a state where the treatment liquid contains the redox enzyme and the coenzyme, and
   determining a voltage for treating the treatment liquid and a target current serving as an indicator of completion of treatment of the treatment liquid in accordance with the first relationship and the second relationship.

### Advantageous Effects of Invention

The method for controlling a treatment apparatus according to the present disclosure provides advantages from the viewpoint of dealing with a change in a component contained in a treatment target while using a reaction system containing a redox enzyme and a coenzyme.

### Brief Description of Drawings

[Fig. 1] Fig. 1 is a diagram illustrating an example of a treatment apparatus according to an embodiment.
[Fig. 2] Fig. 2 is a flow chart illustrating an example of a method for controlling the treatment apparatus according to the embodiment.
[Fig. 3] Fig. 3 is a graph illustrating a relationship between a voltage value and a current value in Example.
[Fig. 4] Fig. 4 is a graph illustrating a relationship between a voltage value and a difference in the current value in Example.
[Fig. 5] Fig. 5 is a graph illustrating a relationship between the voltage value and a ratio of an amount of a changed current value to an amount of a changed voltage value in Example.
[Fig. 6] Fig. 6 is a graph illustrating a relationship between the voltage value and the current value in Example.
[Fig. 7] Fig. 7 is a graph illustrating a change in the current value over time in Example.

### Description of Embodiments

### (Underlying knowledge forming basis of the present disclosure)

It is considered that a treatment target such as a raw material for producing food is treated by a treatment apparatus using a reaction system containing a redox enzyme and a coenzyme. Regarding operation of such a treatment apparatus, it is considered that a voltage is applied to an electrode, and whether the treatment is completed is determined in accordance with the magnitude of a current generated when the voltage is applied.

Based on the investigation by the present inventor, it is assumed that components in the treatment target are not limited to being the same, and that the components in the treatment target are changed in accordance with the type of the treatment target. Therefore, it is considered that operating conditions of the treatment apparatus, such as a voltage applied to an electrode in the treatment apparatus and the magnitude of a current serving as an indicator of completion of treatment, are adjusted in accordance with the treatment target.

The present inventor performed an intensive investigation and, as a result, newly found that the operation conditions in accordance with the treatment target of the treatment apparatus can be specified on the basis of a relationship between a voltage applied to an electrode and a current value corresponding to the voltage when a treatment liquid stored in the treatment apparatus is in a predetermined state. The present inventor devised the method for controlling a treatment apparatus according to the present disclosure in accordance with the new finding.

### (Embodiments according to the present disclosure)

The embodiments according to the present disclosure will be described below with reference to the drawings. In this regard, all the embodiments described below indicate comprehensive or specific examples. Numerical values, shapes, materials, constituents, arrangement positions of the constituents, connection forms, process conditions, steps, step sequence, and the like described in the following embodiments are exemplifications and are not intended to limit the present disclosure. In this regard, of the constituents in the following embodiments, the constituents not described in the independent claims indicating the highest conceptualization are described as optional constituents. In addition, each drawing is a schematic diagram and is not limited to being precisely drawn.

### (Embodiments)

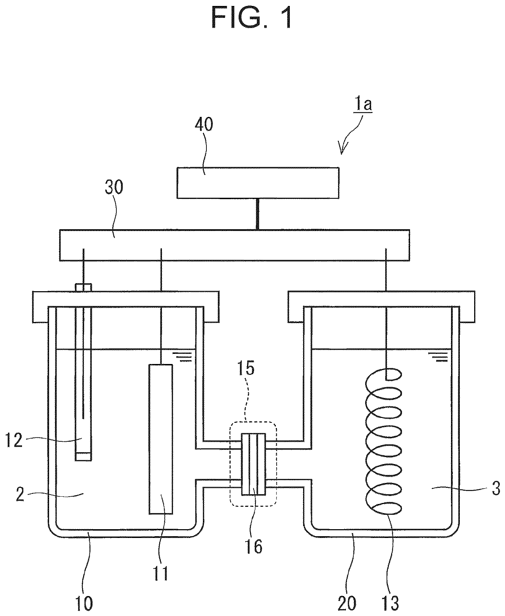

Fig. 1 is a schematic diagram illustrating a treatment apparatus 1a according to an embodiment. In the treatment apparatus 1a, a reaction system containing a redox enzyme and a coenzyme is used. The coenzyme may function as, for example, an electron transport mediator. The treatment apparatus 1a includes a reaction tank 10 (first tank), a first electrode 11, and a second electrode 12. A treatment liquid 2 (first liquid) is stored in the reaction tank 10. The first electrode 11 is disposed in contact with the treatment liquid 2 in the reaction tank 10. The first electrode 11 functions as a working electrode in the treatment apparatus 1a. The second electrode 12 is disposed in contact with the treatment liquid 2 in the reaction tank 10. The second electrode 12 functions as, for example, a reference electrode in a three-electrode method. The treatment apparatus 1a further includes, for example, a third electrode 13 serving as a counter electrode.

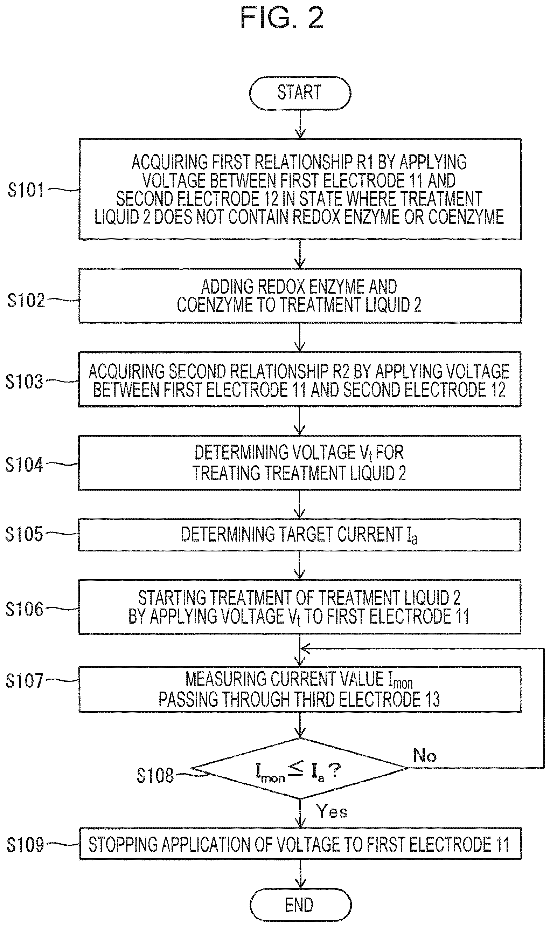

Fig. 2 is a flow chart illustrating a method for controlling the treatment apparatus 1a. In Step S101, a voltage where the second electrode 12 is used as a standard is applied to the first electrode 11 in a state where the treatment liquid 2 does not contain the redox enzyme or the coenzyme, and a voltage for passing a current through the third electrode 13 in the opposite direction, where the current has the same absolute value as the current passing through the first electrode 11, is applied in accordance with the three-electrode method. Hereafter, application of a voltage between the first electrode 11 and the second electrode 12 means the three-electrode method including application of a voltage to the third electrode 13, unless otherwise specified. In such an instance, a first relationship R1 between the first voltage value and the first current value is acquired. The first voltage value is a value of a voltage between the first electrode 11 and the second electrode 12. The first current value is a current value corresponding to the first voltage value. The first current value is, for example, a measurement value of a current in the third electrode 13. In Step S101, for example, the first relationship R1 is acquired by measuring a current in the third electrode 13 while the first voltage value is changed at a predetermined rate. For example, in Step S101, the first relationship R1 is acquired by measuring a current in the third electrode 13 while a voltage between the first electrode 11 and the second electrode 12 is swept at a predetermined rate in a predetermined voltage range.

As illustrated in Fig. 1, the treatment apparatus 1a includes a voltage application apparatus 30. The voltage application apparatus 30 is, for example, a potentiostat. In such an instance, the first electrode 11, the second electrode 12, and the third electrode 13 are electrically coupled to a working electrode terminal, a reference electrode terminal, and a counter electrode terminal, respectively, of the potentiostat.

As illustrated in Fig. 1, the treatment apparatus 1a further includes a controller 40. The controller 40 is connected to the voltage application apparatus 30 so as to be capable of controlling the voltage application apparatus 30. The controller 40 is, for example, a digital computer storing an executable control program for the voltage application apparatus 30. In Step S101, for example, the controller 40 acquires data indicating the first current value from the voltage application apparatus 30. The controller 40 correlates the data with data indicating the first voltage value so as to produce data indicating the first relationship R1 and store the data indicating the first relationship R1 in predetermined memory.

The first relationship R1 may include a relationship between the first voltage value and the first current value acquired by changing the first voltage value at a different rate in a specific voltage range. In such an instance, in Step S101, the first relationship R1 may be acquired by measuring a current in the third electrode 13 while a voltage between the first electrode 11 and the second electrode 12 is swept at a different rate in a predetermined voltage range.

The first relationship R1 may include a relationship between the first voltage value and the first current value in a voltage range corresponding to the treatment target in the treatment liquid 2. For example, when the treatment target is albumen, the first relationship R1 may include a relationship between the first voltage value and the first current value in the voltage range of 0.9 V to 1.1 V. In the first relationship R1, for example, the first current values corresponding to the first voltage values of 0.9 V, 1.0 V, and 1.1 V are specified.

Next, in Step S102, a redox enzyme and a coenzyme are added to the treatment liquid 2. The redox enzyme and the coenzyme may be manually added or automatically added.

Neither the redox enzyme nor the coenzyme is limited to a specific enzyme. When the treatment liquid 2 contains glucose, the redox enzyme is, for example, glucose dehydrogenase (GDH). In addition, the coenzyme is, for example, nicotinamide adenine dinucleotide (NAD).

Next, in Step S103, a voltage is applied between the first electrode 11 and the second electrode 12. In other words, a voltage is applied between the first electrode 11 and the second electrode 12 in a state where the treatment liquid 2 contains the redox enzyme and the coenzyme. In such an instance, a second relationship R2 between a second voltage value and a second current value is acquired. The second voltage value is a value of a voltage between the first electrode 11 and the second electrode 12. The second current value is a current value corresponding to the second voltage value. The second current value is, for example, a measurement value of a current in the third electrode 13. In Step S103, for example, the second relationship R2 is acquired by measuring a current in the third electrode 13 while the second voltage value is changed at a predetermined rate. For example, in Step S 103, the second relationship R2 is acquired by measuring a current in the third electrode 13 while a voltage between the first electrode 11 and the second electrode 12 is swept in a predetermined voltage range at a predetermined rate.

In Step S103, for example, the controller 40 acquires data indicating the second current value from the voltage application apparatus 30. The controller 40 correlates the data with data indicating the second voltage value so as to produce data indicating the second relationship R2 and store the data indicating the second relationship R2 in predetermined memory.

Next, in Step S 104 and Step S 105, a voltage Vt for treating the treatment liquid 2 and a target current Iₐ serving as an indicator of completion of treatment of the treatment liquid 2 are determined in accordance with the first relationship R1 and the second relationship R2.

A voltage that is desirably applied to an electrode and the magnitude of a current serving as an indicator of completion of treatment in the treatment apparatus 1a may be changed in accordance with a change in the component of the treatment target contained in the treatment liquid 2. Since the voltage Vt and the target current Iₐ are determined in accordance with the first relationship R1 and the second relationship R2, the treatment apparatus 1a is readily operated under a predetermined condition in accordance with a change in the component of the treatment target.

In Step S104 and Step S105, for example, the controller 40 performs a predetermined calculation using the data indicating the first relationship R1 and the data indicating the second relationship R2 so as to determine the voltage Vt and the target current Iₐ.

Methods for determining the voltage Vt and the target current Iₐ are not limited to specific methods provided that the methods are in accordance with the first relationship R1 and the second relationship R2. For example, in Step S104, the voltage Vt is determined on the basis of a difference ΔI₁₂ and a ratio ΔI₂/ΔV₂. The difference ΔI₁₂ is a difference between the first current value in the first relationship R1 and the second current value in the second relationship R2. This difference is a difference between the first current value and the second current value corresponding to the first voltage value and the second voltage value, respectively, when the first voltage value in the first relationship R1 is equal to the second voltage value in the second relationship R2. The ratio ΔI₂/ΔV₂ is a ratio of an amount of the changed second current value to an amount of the changed second voltage value ΔV₂. The treatment time of the treatment liquid 2 tends to be decreased by the difference ΔI₁₂ being taken into consideration when the voltage Vt is determined. The treatment liquid 2 tends to be stably treated by the ratio ΔI₂/ΔV₂ being taken into consideration when the voltage Vt is determined.

In Step S104, for example, a voltage range in which the difference ΔI₁₂ is greater than or equal to a predetermined lower limit value is specified. In addition, a voltage range in which the ratio ΔI₂/ΔV₂ is less than or equal to a predetermined upper limit value is specified. The voltage Vt is determined in the range in which these voltage ranges overlap with each other.

In Step S105, for example, the target current Iₐ is determined in accordance with the first current value corresponding to the voltage Vt determined in Step S104 in the first relationship R1. According to such a method, the target current Iₐ corresponding to the voltage Vt can be determined.

For example, when the first relationship R1 includes a relationship between the first voltage value and the first current value acquired by changing the first voltage value at a different rate in a specific voltage range, the current value corresponding to the voltage Vt is determined for each change rate of the first voltage value. Thereafter, the current value when the change rate of the first voltage value is zero may be determined by using an approximate formula denoting a relationship between the resulting current value and the change rate of the first voltage value, and the resulting current value may be determined to be the target current Iₐ.

For example, when the first relationship R1 includes a relationship between the first voltage value and the first current value in a voltage range corresponding to the treatment target in the treatment liquid 2, the target current Iₐ may be determined by using an approximate formula of the relationship between the first voltage value and the first current value. For example, the first current value corresponding to the voltage Vt may be specified from the approximate formula, and the resulting first current value may be determined to be the target current Iₐ.

Next, as illustrated in Steps S106, S107, S108, and S109, the treatment liquid is treated by applying the voltage Vt for treating the treatment liquid 2. In addition, application of the voltage Vt is stopped when a current value generated by application of the voltage Vt is less than or equal to the target current Iₐ.

In Step S106, treatment of the treatment liquid 2 is started by applying the voltage Vt to the first electrode 11. For example, the controller 40 controls the voltage application apparatus 30 so that the voltage Vt is applied to the first electrode 11. Subsequently, in Step S107, a value Iₘₒₙ of a current passing through the third electrode 13 is measured at a predetermined timing after starting treatment of the treatment liquid 2. For example, the controller 40 acquires data indicating the current value Iₘₒₙ. Next, in Step S108, it is determined whether the current value Iₘₒₙ is less than or equal to the target current Iₐ. For example, the controller 40 performs determination in Step S108.

When the determination result in Step S108 is No, the procedure returns to Step S107, the value Iₘₒₙ of a current passing through the third electrode 13 is measured again at a predetermined timing, and determination in Step S108 is performed.

When the determination result in Step S108 is Yes, the procedure advances to Step S109, and application of the voltage to the first electrode 11 is stopped so that a treatment series is completed.

As illustrated in Fig. 1, the treatment apparatus 1a further includes, for example, a second tank 20. A second liquid 3 is stored in the second tank 20. The third electrode 13 is disposed, for example, in contact with the second liquid 3 in the second tank 20.

Each of the treatment liquid 2 and the second liquid 3 contains, for example, a phosphate buffer. In such a configuration, the pH of the treatment liquid 2 and the second liquid 3 can be prevented from rapidly changing, and accordingly, the treatment liquid 2 tends to be stably treated.

The treatment apparatus 1a further includes, for example, a membrane 16. The membrane 16 separates the inside of the reaction tank 10 from the inside of the second tank 20. For example, the membrane 16 hinders the redox enzyme and the coenzyme contained in the treatment liquid 2 from passing through and, in addition, has ionic conductivity. In such a configuration, the energy efficiency of the treatment apparatus 1a tends to be increased.

For example, when the treatment target contained in the treatment liquid 2 is D-glucose, the redox enzyme is GDH, and the coenzyme is NAD, a reaction represented by Formula (1) below occurs in the treatment apparatus 1a. GDH serving as a redox enzyme is involved in the reaction.

D-glucose + NAD⁺ → D-glucono-1,5-lactone + NADH + H⁺ Formula (1)

In the treatment apparatus 1a, reduced nicotinamide adenine dinucleotide (NADH) generated in accordance with Formula (1) is oxidized by the first electrode 11 serving as a working electrode. Consequently, oxidized nicotinamide adenine dinucleotide (NAD⁺) is generated. Since NAD⁺ is generated by continuous oxidation of NADH, glucose can be continuously decomposed even when a large amount of NAD is not contained in the treatment liquid 2.

The membrane 16 hinders the redox enzyme and the coenzyme from passing through. Consequently, the coenzyme present in the reaction tank 10 remains in the reaction tank 10 and is not readily introduced into the second tank 20. As a result, the treatment apparatus 1a tends to prevent the generation of a reduced coenzyme such as NADH due to reduction of an oxidized coenzyme such as NAD⁺ by the third electrode 13. Accordingly, the treatment apparatus 1a decomposes glucose while continuously oxidizing nicotinamide adenine dinucleotide and provides advantages from the viewpoint of energy efficiency. Since the membrane 16 has ionic conductivity, for example, ions of a predetermined type pass through the membrane 16 to maintain electrical neutrality of the treatment liquid 2 and the second liquid 3.

The material for forming the first electrode 11 is not limited to a specific material. The surface of the first electrode 11 is formed of, for example, platinum. In such a configuration, the reduced coenzyme tends to be directly oxidized by the first electrode 11, and the treatment target in the treatment liquid 2 may be efficiently treated. The material constituting the surface of the first electrode 11 may be gold (Au), a carbon material such as glassy carbon, graphite, or boron-doped diamond, or indium tin oxide (ITO).

The shape of the first electrode 11 is not limited to a specific shape. The first electrode 11 may be a linear shape, a tabular shape, a mesh, or a fiber assembly. From the viewpoint of efficiency of treatment of the treatment liquid 2, it is advantageous that the first electrode 11 have a large surface area.

The material for forming the third electrode 13 is not limited to a specific material. The surface of the third electrode 13 is formed of, for example, platinum. In such a configuration, since electrons are readily released from the third electrode 13 to maintain electrical neutrality of the treatment liquid 2 and the second liquid 3 in response to oxidization of the coenzyme, the treatment liquid 2 may be efficiently treated. The material constituting the surface of the third electrode 13 may be gold (Au), a carbon material such as glassy carbon, graphite, or boron-doped diamond, or indium tin oxide (ITO).

The shape of the third electrode 13 is not limited to a specific shape. The third electrode 13 may be a linear shape, a tabular shape, a mesh, or a fiber assembly. From the viewpoint of efficiency of treatment of the treatment liquid 2, it is advantageous that the third electrode 13 have a large surface area.

The material for forming the membrane 16 is not limited to a specific material provided that the material hinders the redox enzyme and the coenzyme from passing through and, in addition, has ionic conductivity. The membrane 16 contains, for example, a polymer having a perfluoro side chain including a sulfonic acid group. In such a configuration, ions such as protons or the like readily pass through the membrane 16, and the treatment liquid 2 may be efficiently treated. Known examples of such a polymer include Nafion (registered trademark). The material for forming the membrane 16 may be porous glass or porous silicon.

As illustrated in Fig. 1, the treatment apparatus 1a includes, for example, a connection portion 15. The connection portion 15 is formed by, for example, the membrane 16 being disposed between a tube-shaped portion protruding from the side portion of the reaction tank 10 and a tube-shaped portion protruding from the side portion of the second tank 20.

In the treatment apparatus 1a, the second tank 20 and the membrane 16 may be omitted, and the third electrode 13 may be disposed in contact with the treatment liquid 2 in the reaction tank 10.

The treatment liquid 2 may contain food or a raw material for producing food. A component serving as the treatment target is contained in the food or the raw material for producing the food, and specific examples of the component include saccharides such as glucose and alcohols such as ethanol.

### EXAMPLES

The method for controlling a treatment apparatus according to the present disclosure will be described in more detail with reference to Examples. In this regard, the method for controlling a treatment apparatus and the treatment apparatus according to the present disclosure are not limited to Examples below.

An apparatus corresponding to the treatment apparatus 1a illustrated in Fig. 1 was prepared in Example. In the apparatus, an electrode in which a platinum wire having a diameter of 0.5 mm and a length of 23 cm was wound into a coil was used as a first electrode serving as a working electrode. A silver/silver chloride (Ag/AgCl) electrode was used as a second electrode serving as a reference electrode. An electrode in which a platinum wire having a diameter of 0.5 mm and a length of 23 cm was wound into a coil was used as a third electrode serving as a counter electrode. OctoStat30 provided by Ivium Technologies B.V. was used as a potentiostat, and a three-electrode cell was formed by electrically coupling the working electrode, the reference electrode, and the counter electrode to a working electrode terminal, a reference electrode terminal, and a counter electrode terminal, respectively, of the potentiostat. An ion-exchange membrane Nafion 117 provided by MTI Corporation was used as a membrane for separating the inside of the first tank from the inside of the second tank.

Phosphate-buffered saline (PBS) having a pH of 7.4 provided by NIPPON GENE CO., LTD. was stored in the second tank in which the counter electrode was disposed. In the second tank, a portion of the counter electrode was in contact with the PBS. The amount of liquid PBS stored in the second tank was 8.1 milliliters (mL).

Albumin was manually separated from the yolk of a commercially available egg. The resulting albumin was manually agitated by using chopsticks in such a manner that the amount of a highly viscous portion was reduced to the extent of not causing foaming. Thereafter, the albumin was filtered with gauze to remove a solid portion so as to obtain an albumin liquid. Albumin commonly contains about 0.7% glucose on a mass basis at an albumin glucose concentration of about 22 × 10⁻³ [mol/L]. As a treatment liquid, 7.2 mL of the albumin liquid was stored in the first tank serving as the reaction tank.

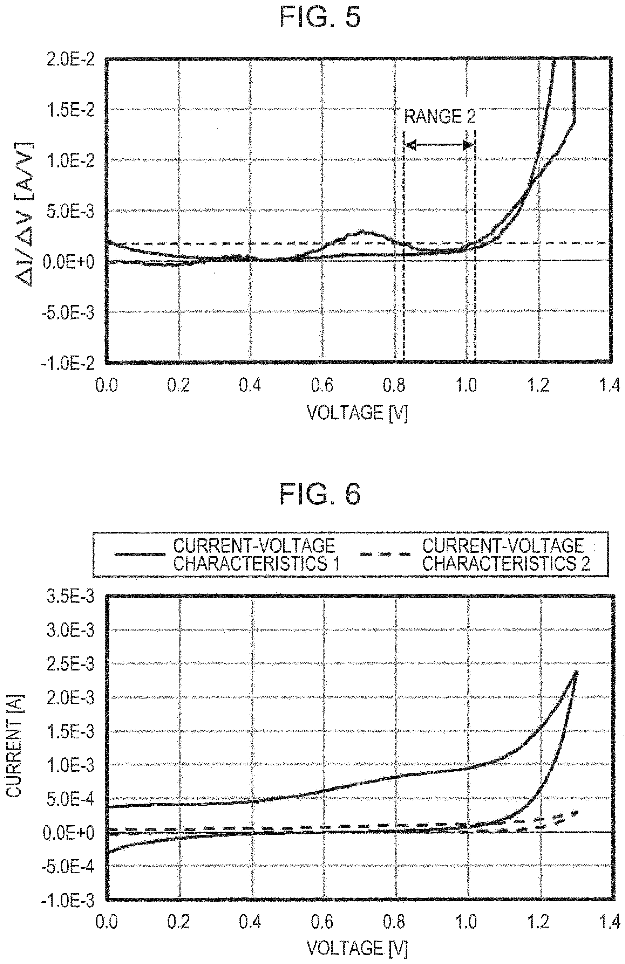

The potential of the reference electrode was taken as a reference potential, a voltage applied to the working electrode was swept in a predetermined range, and the value of a current passing through the counter electrode, the current value corresponding to the voltage value applied to the counter electrode, was measured and stored. A control computer and control software were used to adjust the potential difference between the reference electrode and the working electrode and to measure the value of a current passing through the counter electrode. Electronic data of the current-voltage characteristics 1 and the current-voltage characteristics 2 which indicate a relationship between the value of a voltage applied to the working electrode and the value of a current passing through the counter electrode were stored in the control computer memory. Fig. 6 is a graph illustrating the current-voltage characteristics 1 and the current-voltage characteristics 2. In Fig. 6, the vertical axis represents the value of a current passing through the counter electrode, and the horizontal axis represents the value of a voltage applied to the working electrode.

Regarding acquisition of the current-voltage characteristics 1 and the current-voltage characteristics 2, the range of the voltage applied to the working electrode was -0.6 V to +1.3 V. The voltage applied to the working electrode was started at -0.6 V, increased to +1.3 V at 10-mV intervals, and, thereafter, decreased from +1.3 V to -0.6 V at 10-mV intervals. The current-voltage characteristics 1 were acquired at a sweep rate of 100 mV/sec. On the other hand, the current-voltage characteristics 2 were acquired at a sweep rate of 10 mV/sec.

A NAD solution was prepared by dissolving, at a concentration of 40 × 10⁻³ [mol/L], reduced nicotinamide adenine dinucleotide into the same type of phosphate buffer as the phosphate buffer stored in the second tank. β-Nicotinamide adenine dinucleotide disodium (reduced form) provided by NACALAI TESQUE, INC. was used as reduced nicotinamide adenine dinucleotide. A GDH solution was prepared by dissolving, at a concentration of 80 [unit/mL], glucose dehydrogenase into the same type of phosphate buffer as the phosphate buffer stored in the second tank. Glucose dehydrogenase (GDH) provided by FUJIFILM Wako Pure Chemical Corporation was used as glucose dehydrogenase. Addition of 0.8 mL of NAD solution and 0.1 mL of GDH solution to the treatment liquid stored in the first tank was performed.

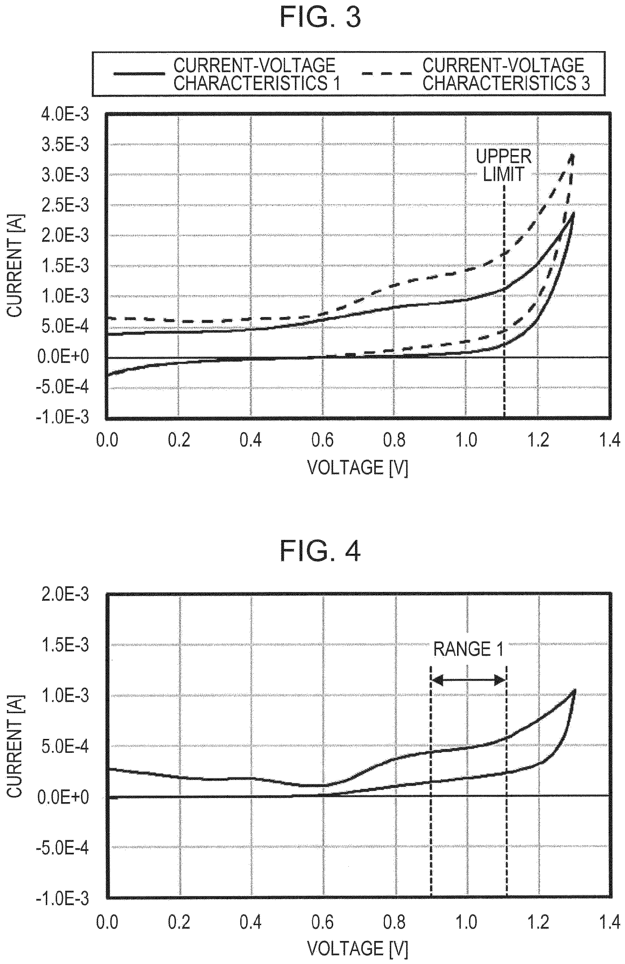

Thereafter, a voltage applied to the working electrode was swept in a predetermined range under the same condition as the voltage range and the sweep rate when the current-voltage characteristics 1 were acquired, and the value of a current passing through the counter electrode, the current value corresponding to the voltage value applied to the counter electrode, was measured. The thus acquired current-voltage characteristics 3 were then stored. Fig. 3 is a graph illustrating the current-voltage characteristics 1 and the current-voltage characteristics 3. In Fig. 3, the vertical axis represents the value of a current passing through the counter electrode, and the horizontal axis represents the value of a voltage applied to the working electrode.

As illustrated in Fig. 3, when the voltage applied to the working electrode was increased to higher than +1.1 V, the current passing through the counter electrode rapidly increased. The upper limit value of the voltage applied to the working electrode in the apparatus according to Example was determined to be +1.1 V. This determination was performed in consideration of circumstances described in (I) and (II) below.
(I) A voltage higher than +1.1 Vis close to a voltage at which electrolysis of water occurs at the working electrode, as a result of which O₂ is generated.
(II) It is difficult in principle to consider that the amount of electrons provided to the working electrode by NAD rapidly increases relative to the voltage.

Fig. 4 is a graph illustrating a relationship between the voltage applied to the working electrode and the difference ΔI₁₂ which is the difference between the current value of the current-voltage characteristics 1 and the current value of the current-voltage characteristics 3. In Fig. 4, the vertical axis represents the value of a current passing through the counter electrode, and the horizontal axis represents the value of a voltage applied to the working electrode. The difference ΔI₁₂ is considered to be a current in accordance with the electrons provided to the working electrode by NAD. Accordingly, it is understood that treatment of the treatment liquid tends to be shortened with an increase in the difference ΔI₁₂. In accordance with the upper limit value of the voltage applied to the working electrode being determined to be +1.1 V, as illustrated in Fig. 3, the selection range of the voltage applied to the working electrode was determined to be a range 1 of +0.9 V to +1.1 V, as illustrated on the graph in Fig. 4.

Fig. 5 is a graph illustrating a relationship between the voltage applied to the working electrode and a ratio ΔI₂/ΔV₂ of an amount of the changed current value ΔI₂ to an amount of the changed voltage value ΔV₂ with respect to the current-voltage characteristics 3. In Fig. 5, the vertical axis represents the ratio ΔI₂/ΔV₂, and the horizontal axis represents the voltage applied to the working electrode. When the ratio ΔI₂/ΔV₂ is large, there is a possibility of the treatment of the treatment liquid being unstable. Accordingly, the upper limit value of the ratio ΔI₂/ΔV₂ was set to be 1.7 × 10⁻³ [A/V], and the selection range of the voltage applied to the working electrode was determined to be a range 2 of +0.85 V to +1.05 V, as illustrated on the graph in Fig. 5.

The selection range of the voltage applied to the working electrode to treat the treatment liquid was determined to be a range of +0.9 V to +1.05 V in which the range 1 overlapped the range 2. Subsequently, the voltage Vt applied to the working electrode to treat the treatment liquid in the apparatus according to Example was determined to be +1.0 V.

Next, the target current Iₐ serving as an indicator of completion of treatment of the treatment liquid was determined as described below. Regarding the current-voltage characteristics 1, each time the voltage applied to the working electrode was increased or decreased, a current value corresponding to the voltage Vt was specified, and a current value α that was the average value of the current values was determined. In Example, the current value α was 5.1 × 10⁻⁴ A.

Regarding the current-voltage characteristics 2, in the manner akin to that of determining the current value α, each time the voltage applied to the working electrode was increased or decreased, a current value corresponding to the voltage Vt was specified, and a current value β that was the average value of the current values was determined. In Example, the current value β was 6.2 × 10⁻⁵ A. It was assumed that a relationship between the sweep rate of the voltage applied to the working electrode when the current-voltage characteristics 1 and the current-voltage characteristics 2 were acquired and each of the current value α and the current value β was a linear relationship, and a current value when the sweep rate was zero in the linear relationship was determined to be the target current Iₐ. In Example, the target current Iₐ was 1.2 × 10⁻⁵ A.

Next, the treatment of the treatment liquid was started by continuously applying a voltage of +1.0 V between the working electrode and the reference electrode. In addition, the value Iₘₒₙ of the current passing through the counter electrode was measured. The treatment liquid was treated at room temperature (15°C to 27°C). The treatment liquid was agitated by using a stirrer during treatment of the treatment liquid.

When checking that the value Iₘₒₙ of the current passing through the counter electrode became less than or equal to the target current Iₐ, application of the voltage to the working electrode was stopped.

The treatment liquid was sampled at a predetermined timing during continuous application of the voltage of +1.0 V to the working electrode. Dripping of 50 microliters (µL) of the sampled treatment liquid on a urine glucose inspection portion of a urine test paper New Uriace BT produced by Terumo Corporation was performed, and a change in the color of the urine glucose inspection portion was visually examined so as to check for the presence or absence of decomposed glucose and the degree thereof. Regarding the present urine test paper, it was found that when the concentration of glucose was less than or equal to 0.1 × 10⁻³ [mol/L], the color of the urine glucose inspection portion was almost unchanged even when the treatment liquid was dripped. In addition, it was found that when the concentration of glucose was higher than 0.1 × 10⁻³ [mol/L], the color of the urine glucose inspection portion changed to navy blue, and it was found that the two samples were visually distinguishable.

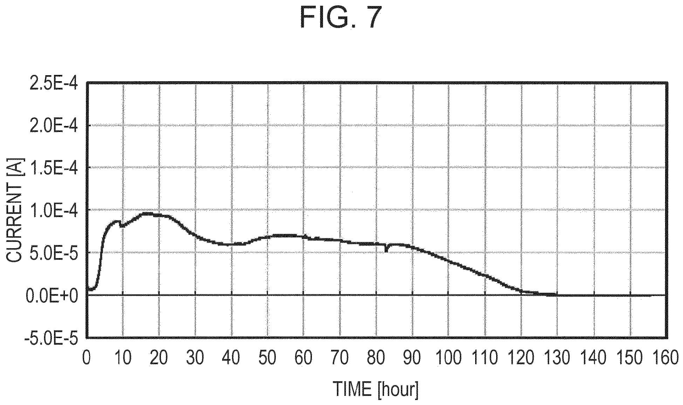

Fig. 7 is a graph illustrating the change in the value Iₘₒₙ of the current passing through the counter electrode over time during treatment of the treatment liquid. In Fig. 7, the vertical axis represents the value of a current passing through the counter electrode, and the horizontal axis represents the elapsed time from starting treatment of the treatment liquid. The current passing through the counter electrode gradually decreased after a lapse of about 90 hours from starting application of a voltage to the working electrode, and the current passing through the counter electrode decreased to less than or equal to the target current Iₐ after a lapse of about 120 hours from starting application of a voltage to the working electrode. As a result of examining the treatment liquid sampled every 50 hours after starting application of a voltage to the working electrode by using urine test paper, glucose was clearly detected in the treatment liquid after a lapse of 50 hours and after a lapse of 100 hours from starting application of a voltage. On the other hand, the treatment liquid after a lapse of 150 hours from starting application of a voltage to the working electrode did not change the color of the urine test paper, and it was found that glucose in the treatment liquid was decomposed to a concentration lower than or equal to the detection limit.

### Industrial Applicability

An electrochemical reaction apparatus according to the present disclosure is useful for electrochemical decomposition of, for example, glucose.

### Reference Signs List

1a treatment apparatus
2 treatment liquid
10 reaction tank
11 first electrode
12 second electrode
13 third electrode

## Claims

1. A method for controlling a treatment apparatus using a reaction system containing a redox enzyme and a coenzyme,
the treatment apparatus including a reaction tank storing a treatment liquid, a first electrode disposed in contact with the treatment liquid in the reaction tank, and a second electrode disposed in contact with the treatment liquid in the reaction tank,
the method comprising:
acquiring a first relationship between a first voltage value and a first current value corresponding to the first voltage value by applying a voltage between the first electrode and the second electrode in a state where the treatment liquid does not contain the redox enzyme or the coenzyme;
acquiring a second relationship between a second voltage value and a second current value corresponding to the second voltage value by applying a voltage between the first electrode and the second electrode in a state where the treatment liquid contains the redox enzyme and the coenzyme; and
determining a voltage for treating the treatment liquid and a target current serving as an indicator of completion of treatment of the treatment liquid in accordance with the first relationship and the second relationship.

2. The method for controlling a treatment apparatus according to claim 1, wherein
the voltage for treating the treatment liquid is determined in accordance with a difference between the first current value and the second current value and with a ratio of an amount of the changed second current value to an amount of the changed second voltage value.

3. The method for controlling a treatment apparatus according to claim 1 or claim 2, wherein
the target current is determined in accordance with the first current value corresponding to the voltage for treating the treatment liquid in the first relationship.

4. The method for controlling a treatment apparatus according to any one of claims 1 to 3, wherein
the first relationship includes a relationship between the first voltage value and the first current value acquired by changing the first voltage value at a different rate in a predetermined voltage range.

5. The method for controlling a treatment apparatus according to any one of claims 1 to 3, wherein
the first relationship includes a relationship between the first voltage value and the first current value in a voltage range corresponding to the treatment target.

6. The method for controlling a treatment apparatus according to any one of claims 1 to 5,
the method comprising:
treating the treatment liquid by applying the voltage for treating the treatment liquid, and
stopping application of the voltage when a current value generated by application of the voltage is less than or equal to the target current.

7. The method for controlling a treatment apparatus according to any one of claims 1 to 6, wherein
the treatment liquid contains food or a raw material for producing food.

8. A treatment apparatus using a reaction system containing a redox enzyme and a coenzyme, the treatment apparatus comprising:
a reaction tank storing a treatment liquid;
a first electrode disposed in contact with the treatment liquid in the reaction tank; and
a second electrode disposed in contact with the treatment liquid in the reaction tank,
wherein
the treatment apparatus is configured to:
acquire a first relationship between a first voltage value and a first current value corresponding to the first voltage value by applying a voltage between the first electrode and the second electrode in a state where the treatment liquid does not contain the redox enzyme or the coenzyme,
acquire a second relationship between a second voltage value and a second current value corresponding to the second voltage value by applying a voltage between the first electrode and the second electrode in a state where the treatment liquid contains the redox enzyme and the coenzyme, and
determine a voltage for treating the treatment liquid and a target current serving as an indicator of completion of treatment of the treatment liquid in accordance with the first relationship and the second relationship.
